# EUROPEAN PATENT APPLICATION

(11) **EP 2 810 642 A1**
(43) Date of publication of application: **10.12.2014**
(21) Application number: 13179674.0
(22) Date of filing: 08.08.2013
(51) Int. Cl.: A61K 9/127, A61K 36/185, A61K 36/28, A61K 36/33, A61K 9/00

(54) **Chitosan-modified ethosome structure**

(30) Priority: 05.06.2013 TW 102210593
(71) Applicant: Lee, Chin-Tung, New Taipei City 231 (TW)
(72) Inventor: Lee, Chin-Tung, 231 New Taipei City (TW); Chen, Po-Liang, 114 Taipei City (TW)
(74) Representative: Viering, Jentschura & Partner Patent- und Rechtsanwälte

(57) **Abstract**

A chitosan-modified ethosome structure **10** having property of targeting is provided. The chitosan-modified ethosome structure **10** includes a first liposome shell **100** and a second liposome shell **200.** The external radius of the first liposome shell **100** is smaller than the internal radius of the second liposome shell **200,** and the second liposome shell **200** encloses the first liposome shell **100.** In addition, a first space **50** is defined in the inside of the first liposome shell **100,** and a second space **150** is defined between the inside surface of the second liposome shell **200** and the outside surface of the first liposome shell **100.** A plurality of ring-shaped structures **105** is formed on the outside surface of the second liposome shell **200.** Each ring-shaped structure **105** is consisted of algae sugar and beta-cyclodextrin. The first space **50** is for containing a first active substance **300,** and the second space **150** is for containing a second active substance **400.** The chitosan-modified ethosome structure **10** contains active substances with different effects, such that improves the storage and transportation of multi-active substances.

## Description

### BACKGROUND

### Technical Field

The present disclosure relates generally to ethosome structure, and more generally to chitosan-modified ethosome structure for enclosing Mexican yam extract, artichoke extract, cactus extract, and other active substances.

### Related Art

The surface of human skin has an external barrier composed of cuticle and epidermis. Due to the obstruction of the skin barrier, most active substances for external use hardly pass through the skin at all. Consequently, the absorption and distribution of the active substances is restricted significantly.

### SUMMERY

The disclosure is directed to a specially-modified ethosome having the property of targeting. It is capable of carrying active substances through the skin barrier, into the dermis and the hypodermis. It is also capable of entering the systemic circulation through the hypodermis microvascule, and being released on arrival at a point of a target organ or tissue. For pharmacokinetics in transcutaneous absorption, the absorption distribution is improved substantially, enhancing the effect of the active substances.

The present disclosure provides a chitosan-modified ethosome structure which is primarily composed of Phosphatidylcholine (PC), Phosphatidylserine (PS) and phosphatidylinositol (PI). It is capable of enclosing and carrying a plurality of micronized particles of Mexican yam extract, Artichoke extract, and cactus extract. By using ethanol and phospholipid which is the primary composition of cell membrane, the ethosome is capable of enclosing the Mexican yam extract (Mexican yam root extract). Then the ethosome is transported to dermis and hypodermis microvascular by progressive nutrition diffusion technology (PNDT), arriving a specific action point of thymus through the systemic circulation to stimulate breast development and the proliferation of collagen and fibroblasts, so as to result in tightening and lifting of the breast. Through the above-mentioned techniques, the ethosome can be used for skin care if carrying artichoke extract, and can be used for reducing body fat if carrying cactus extract.

The chitosan-modified ethosome structure includes a first liposome shell and a second liposome shell. The external radius of the first liposome shell is smaller than the internal radius of the second liposome shell, and the second liposome shell encloses the first liposome shell. In addition, a first space is defined in the inside of the first liposome shell, and a second space is defined between the inside surface of the second liposome shell and the outside surface of the first liposome shell. A plurality of ring-shaped structures is formed on the outside surface of the second liposome shell. Each ring-shaped structure is consisted of algae sugar and beta-cyclodextrin. The height along the axial direction of each ring-shaped structure is smaller than the diameter of the ring-shaped structure.

Another aspect of the disclosure is the first liposome shell and the second liposome shell of the chitosan-modified ethosome structure are respectively composed from chitosan-modified ethosome, and the chitosan-modified ethosome includes a plurality of phospholipid molecules and a plurality of ethanol molecules.

Another aspect of the disclosure is the first space of the chitosan-modified ethosome structure is for containing at least a first active substance, and the second space is for containing at least a second active substance.

Another aspect of the disclosure is each of the ring-shaped structure is for containing the first active substance or the second active substance.

Another aspect of the disclosure is that the first active substance is not the same as the second active substance.

Another aspect of the disclosure is that the first active substance is selected from a group consisting of Mexican yam extract, artichoke extract and cactus extract.

Another aspect of the disclosure is that the second active substance is selected from a group consisting of Mexican yam extract, artichoke extract and cactus extract.

Another aspect of the disclosure is that the phospholipid molecules of the first liposome shell respectively has at least a phospholipid hydrophilic group and at least a phospholipid hydrophobic group, the phospholipid hydrophilic group is located at the inside of the first liposome shell so as to enclose the first active substances from outside to inside, the phospholipid hydrophobic group is located at the outside of the first liposome shell so as to enclose the second active substances from inside to outside.

Another aspect of the disclosure is that the ethanol molecules of the first liposome shell respectively has at least one ethanol hydroxyl and at least one hydrocarbon chain, the ethanol hydroxyl is located at the inside of the first liposome shell so as to enclose the first active substances from outside to inside, the hydrocarbon chain is located at the outside of the first liposome shell so as to enclose the second active substances from inside to outside.

Another aspect of the disclosure is that the phospholipid molecules of the second liposome shell respectively has at least a phospholipid hydrophilic group and at least a phospholipid hydrophobic group, the phospholipid hydrophilic group is located at the inside of the second liposome shell so as to enclose the first active substances from outside to inside, the phospholipid hydrophobic group is located at the outside of the second liposome shell so as to enclose the second active substances from inside to outside.

Another aspect of the disclosure is that the ethanol molecules of the second liposome shell respectively has at least one ethanol hydroxyl and at least one hydrocarbon chain, the ethanol hydroxyl is located at the inside of the second liposome shell so as to enclose the first active substances from outside to inside, the hydrocarbon chain is located at the outside of the second liposome shell so as to enclose the second active substances from inside to outside.

It is to be understood that both the foregoing general description and the following detailed description presented below are intended to provide an overview or framework for understanding the nature and character of the disclosure as it is claimed. The accompanying drawings are included to provide a further understanding of the disclosure, and are incorporated into and constitute a part of this specification. The drawings illustrate various embodiments of the disclosure and together with the description serve to explain the principles and operations of the disclosure.

### BRIEF DESCRIPTION OF THE DRAWINGS

FIG. 1 is a partial sectional view of the chitosan-modified ethosome structure of the present disclosure;

FIG. 2 is another partial sectional view of the chitosan-modified ethosome structure of the present disclosure;

FIG. 3 is a partial sectional view of the chitosan-modified ethosome structure of the present disclosure which store active substances;

FIG. 4A is a schematic diagram of phospholipid molecules of ethosome;

FIG. 4B is a schematic diagram of ethanol molecules of ethosome;

FIG. 4C is a schematic diagram of first active substance;

FIG. 4D is a schematic diagram of second active substance.

### DETAILED DESCRIPTION

Reference is now made in detail to various embodiments of the disclosure, examples of which are illustrated in the accompanying drawings. Whenever possible, the same or like reference numbers and symbols are used throughout the drawings to refer to the same or like parts. The drawings are not necessarily to scale, and one skilled in the art will recognize where the drawings have been simplified to illustrate the key aspects of the disclosure.

The claims as set forth below are incorporated into and constitute part of this Detailed Description.

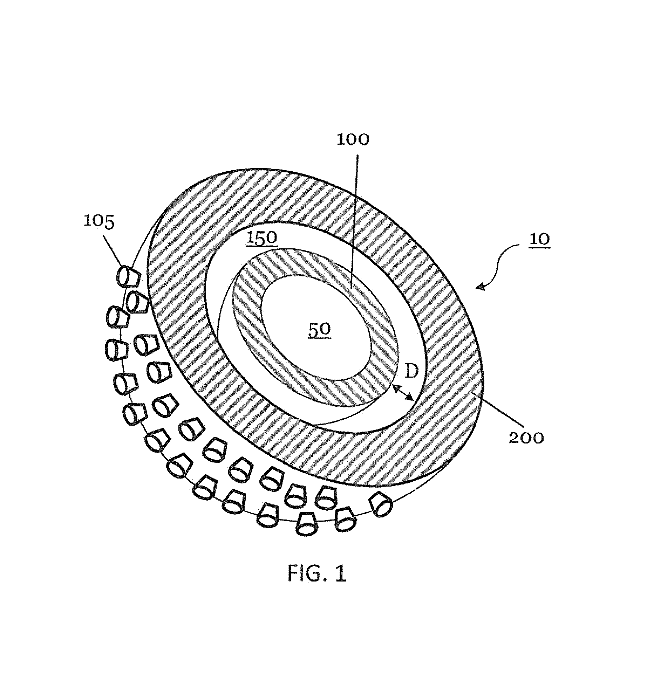

Please refer to Fig. 1, which illustrates a chitosan-modified ethosome structure 10. Chitosan-modified ethosome structure 10 is primarily consisted of a first liposome shell 100 and a second liposome shell 200.

Both of first liposome shell 100 and second liposome shell are hollow spherical structured, and first liposome shell 100 is located inside of the second liposome shell 200. A first space 50 for containing specific active substances is defined in the inside of first liposome shell 100. The external radius of the first liposome shell 100 is smaller than the internal radius of the second liposome shell 200, the difference between the external radius of the first liposome shell 100 and the internal radius of the second liposome shell 200 is defined as D. A second space 150 is defined between the inside surface of the second liposome shell 200 and the outside surface of the first liposome shell 100.

In addition, a plurality of ring-shaped structures 105 is formed on the outside surface of the second liposome shell 200. The height along the axial direction of each ring-shaped structure 105 is smaller than the diameter thereof, so that the ring-shaped structure 105 has a substantially flat appearance. The surrounding space of each ring-shaped structure 105 can be used to carry the active substances, so as to elevate the amount of the active substances being carried by chitosan-modified ethosome structure 10. By modifying the quantity of ring-shaped structures 105, the amount of the active substances being carried by chitosan-modified ethosome structure 10 can be finely adjusted. Each ring-shaped structure 105 is consisted of algae sugar and beta-cyclodextrin.

First space 50 and second apace 150 are capable of being used to contain different active substances. Therefore, chitosan-modified ethosome 10 is capable of enclosing different active substances to be transported and released. The composition of first liposome shell 100 and second liposome shell 200 as well as the active substances contained in the first space 50 and second space 150 will be discussed in following paragraphs.

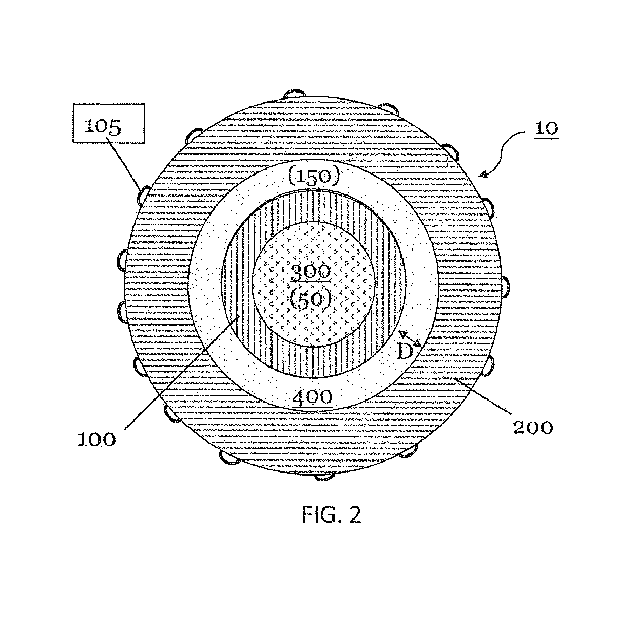

Please refer to Fig. 2, Fig. 3, and Figs. 4A-4D, in which first liposome shell 100 and second liposome shell 200 are respectively an ethosome with a hollow spherical structure. The ethosome primarily includes a plurality of phospholipid molecules 500 and a plurality of ethanol molecules 600. The ethosome is different from the conventional phytosome. In the ethosome, the contained phospholipid molecules 500 are not combined with the enclosed active substances. While in the conventional phytosome, the phospholipid molecules will be combined with the enclosed active substances and form a new molecule, which remarkably changes the biological property. Consequently, ethosome is more suitable to enclose active substances having different properties than phytosome.

Chitosan-modified ethosome structure 10 encloses different active substances having different properties. First space 50 of first liposome shell 100 is used for containing first active substances 300, the second space 150 are used to contain second active substances 400. Second active substances 400 include a plurality of micronized particles of Mexican yam extract, artichoke extract, and/or cactus extract. First active substances 300 are not exactly the same as the second active substances 400. Chitosan-modified ethosome structure 10 encloses first active substances 300 by first liposome shell 100, second active substances 400 are enclosed between the outside surface of first liposome shell 100 and the inside surface of second liposome shell 200.

Chitosan-modified ethosome structure 10 uses the second space 150 to contain the micronized particles of Mexican yam extract, artichoke extract, and/or cactus extract, or contain other active substances to achieve the function of sustained release and targeting acting.

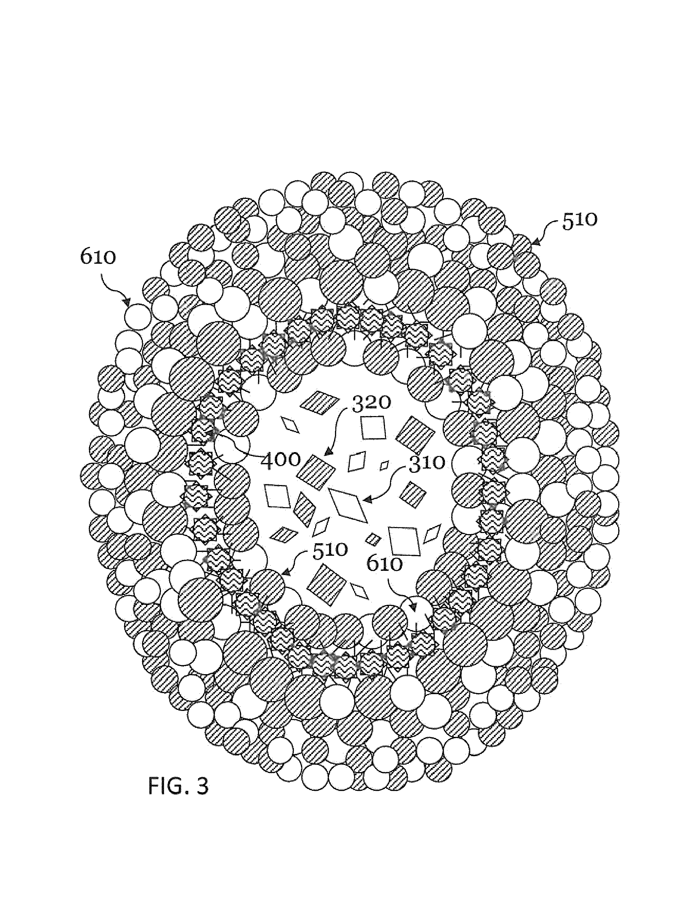

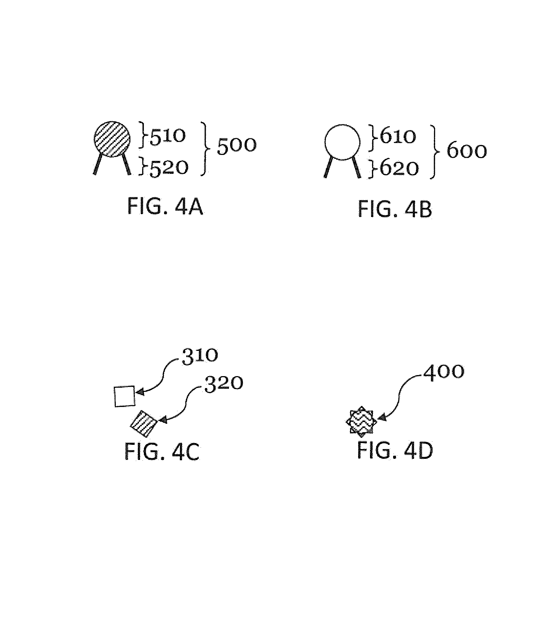

Please refer to Fig. 3 and Figs. 4A-4D., in which first liposome shell 100 is composed of phospholipid molecules 500 and ethanol molecules 600. Each phospholipid molecule 500 has at least a phospholipid hydrophilic group 510 and at least a phospholipid hydrophobic group 520. Phospholipid hydrophilic group 510 is located at the inside of first liposome shell 100 so as to enclose first active substances 300 from outside to inside. Phospholipid hydrophobic group 520 is located at the outside of the first liposome shell 100 so as to enclose second active substances 400 from inside to outside. Ethanol molecules 600 of first liposome shell 100 respectively has at least one ethanol hydroxyl 610 and at least one hydrocarbon chain 620. Ethanol hydroxyl 610 is located at the inside of first liposome shell 100 so as to enclose first active substances 300 from outside to inside. Hydrocarbon chain 620 is located at the outside of first liposome shell 100 so as to enclose second active substances 400 from inside to outside.

Second liposome shell 200 is composed ofphospholipid molecules 500 and ethanol molecules 600 as well. Phospholipid molecules 500 of second liposome shell 200 respectively has at least a phospholipid hydrophilic group 510 and at least a phospholipid hydrophobic group 520. Phospholipid hydrophilic group 510 is located at the outside of second liposome shell 200 and phospholipid hydrophobic group 520 is located at the inside of second liposome shell 200, so as to enclose second active substances 400 from outside to inside. Ethanol molecules 600 of second liposome shell 200 respectively has at least one ethanol hydroxyl 610 and at least one hydrocarbon chain 620. Ethanol hydroxyl 610 is located at the inside of second liposome shell 200 and hydrocarbon chain 620 is located at the outside of first liposome shell 100, so as to enclose second active substances 400 from outside to inside.

The previously mentioned first active substance 300 is selected from a group consisted of seaweed extract and micronized particles of Mexican yam extract, artichoke extract and cactus extract, but not limited thereto. In one example, first active substance 300 has a plurality of hydrophilic components 310 and hydrophobic components 320. Due to being enclosed by ethanol hydroxyl 610 of first liposome shell 100, the biological properties of first active substance 300 can remain when being stored and carried.

The previously mentioned second active substance 400 is selected from a group consisted of seaweed extract and micronized particles of Mexican yam extract, artichoke extract and cactus extract, but not limited thereto. Due to being enclosed between phospholipid hydrophobic group 520 located outside of first liposome shell 100 and hydrocarbon chain 620 located inside of second liposome shell 200, the biological properties of second active substance 400 can be remain when being stored and carried.

Chitosan-modified ethosome structure 10 is formed by basically ethosome. First liposome shell 100 and second liposome shell 200 enclose and carry multiple active substances (ie. First active substances 300 and second active substances 400), and keep first active substances 300 and second active substances 400 separated during the storage period. Chitosan-modified ethosome structure 10 can be designed to release first active substance 300 and second active substance 400 at different locations, resulting in slow-release and centralized action.

Chitosan-modified ethosome structure 10 can be modified to have the following functions:
(1) Response to external factors: for example, response to external factors of temperature, pH value, magnetic force and enzyme, so as to regulate the release of first active substance 300 and second active substance 400 and achieve intelligent sensing and releasing.
(2) Targeting: the ethosome modified with antibody or with specific mark can make the multiple active substances be released at specified location to achieve targeting and centralizing action.

To summarize, using chitosan-modified ethosome structure as carrier of active substances of Mexican yam extract, artichoke extract and cactus extract can make the active substances pass through the skin barrier to enter specific areas of the human body, and act.

It will be apparent to those skilled in the art that various modifications and variations can be made to the present disclosure without departing from the spirit and scope of the disclosure. Thus it is intended that the present disclosure cover the modifications and variations of this disclosure provided they come within the scope of the appended claims and their equivalents.

## Claims

1. A chitosan-modified ethosome structure **10** for enclosing and carrying a plurality of micronized particles selected from Mexican yam extract micronized particles, artichoke extract micronized particles, and cactus extract micronized particles, the chitosan-modified ethosome structure comprising:
a first liposome shell **100,** a first space **50** is defined in the inside of the first liposome shell **100;** and
a second liposome shell **200,** enclosing the first liposome shell **100,** a second space 150 is defined between the inside surface of the second liposome shell **200** and the outside surface of the first liposome shell **100,** a plurality of ring-shaped structures **105** is formed on the outside surface of the second liposome shell **200,** each ring-shaped structure **105** is consisted of algae sugar and beta-cyclodextrin, the height along the axial direction of each ring-shaped structure **105** is smaller than the diameter of the ring-shaped structure 105.

2. The chitosan-modified ethosome structure **10** according to claim 1, wherein the first liposome shell 100 and the second liposome shell **200** are respectively composed from chitosan-modified ethosome and the chitosan-modified ethosome comprises a plurality of phospholipid molecules and a plurality of ethanol molecules.

3. The chitosan-modified ethosome structure **10** according to claim 1, wherein the first space 50 is for containing at least a first active substance, and the second space **150** is for containing at least a second active substance.

4. The chitosan-modified ethosome structure **10** according to claim 3, wherein each of the ring-shaped structure 105 is for containing the first active substance or the second active substance.

5. The chitosan-modified ethosome structure **10** according to claims 3 or 4, wherein the first active substance is not the same as the second active substance.

6. The chitosan-modified ethosome structure **10** according to claims 3 or 4, wherein the first active substance is selected from a group consisting of Mexican yam extract, artichoke extract and cactus extract.

7. The chitosan-modified ethosome structure **10** according to claim 3 or 4, wherein the second active substance is selected from a group consisting of Mexican yam extract, artichoke extract and cactus extract.

8. The chitosan-modified ethosome structure **10** according to claim 7, wherein the phospholipid molecules of the first liposome shell respectively has at least a phospholipid hydrophilic group and at least a phospholipid hydrophobic group, the phospholipid hydrophilic group is located at the inside of the first liposome shell **100** so as to enclose the first active substances from outside to inside, the phospholipid hydrophobic group is located at the outside of the first liposome shell **100** so as to enclose the second active substances from inside to outside.

9. The chitosan-modified ethosome structure **10** according to claim 7, wherein the ethanol molecules of the first liposome shell **100** respectively has at least one ethanol hydroxyl and at least one hydrocarbon chain, the ethanol hydroxyl is located at the inside of the first liposome shell 100 so as to enclose the first active substances from outside to inside, the hydrocarbon chain is located at the outside of the first liposome shell **100** so as to enclose the second active substances from inside to outside.

10. The chitosan-modified ethosome structure **10** according to claim 7, wherein the phospholipid molecules of the second liposome shell **200** respectively has at least a phospholipid hydrophilic group and at least a phospholipid hydrophobic group, the phospholipid hydrophilic group is located at the inside of the second liposome shell **200** so as to enclose the first active substances from outside to inside, the phospholipid hydrophobic group is located at the outside of the second liposome shell **200** so as to enclose the second active substances from inside to outside.

11. The chitosan-modified ethosome structure **10** according to claim 7, wherein the ethanol molecules of the second liposome shell **200** respectively has at least a ethanol hydroxyl and at least a hydrocarbon chain, the ethanol hydroxyl is located at the inside of the second liposome shell **200** so as to enclose the first active substances from outside to inside, the hydrocarbon chain is located at the outside of the second liposome shell **200** so as to enclose the second active substances from inside to outside.
